# EUROPEAN PATENT APPLICATION

(11) **EP 1 914 548 A1**
(43) Date of publication of application: **23.04.2008**
(21) Application number: 06122703.9
(22) Date of filing: 20.10.2006
(51) Int. Cl.: G01N 33/50, G01N 33/68

(54) **Method for identifying women with an increased risk of preterm delivery**

(71) Applicant: THE UNIVERSITY OF NEWCASTLE RESEARCH ASSOCIATES LIMITED, Callaghan, NSW 2308 (AU)
(72) Inventor: Smith, Roger, Newcastle New South Wales 2310 (AU)
(74) Representative: Habets, Winand

(57) **Abstract**

This invention relates to sensitive screening methods for the detection of preterm delivery. In particular, this invention is directed to determining an early indication of increased risk of preterm delivery by detecting an abnormal level of a number of biomarkers in particular in the serum, plasma or blood of pregnant women.

## Description

### Field of the Invention

This invention relates to sensitive screening methods for the early detection of preterm delivery. In particular, this invention is directed to determining an early indication of increased risk of preterm delivery by detecting an abnormal level of a number of biomarkers in particular in the serum, plasma or blood of pregnant women.

### Background of the invention

Every year approximately 4.5 million premature babies are born worldwide, and, despite considerable advances in neonatal care, their mortality rate remains high. Moreover, survivors are at risk for long-term handicap, including developmental delay cerebral palsy, blindness, deafness, and chronic lung disease. In particular, preterm neonates account for more than half, and maybe as much as three-quarters of the morbidity and mortality of newborns without congenital anomalies.

Determination of impending preterm births is critical for increasing neonatal survival of preterm infants. Its limited success has been attributed, in part, to the fact that premature parturition is a syndrome caused by multiple pathological processes such as infection, vascular disease, uterine over-distension, and chronic stress.

Although tocolytic agents which can delay delivery were introduced 20 to 30 years ago, there has been only a minor decrease in the incidence of preterm delivery. It has been postulated that the failure to observe a larger reduction in the incidence of preterm births is due to errors in the diagnosis of preterm labour and to the patients conditions being too advanced for tocolytic agents to successfully delay the birth.

Specifically, the diagnosis and the therapeutic treatment of a threat of preterm delivery remain unsolved clinical problems. Rapid identification of patients at risk is difficult. Conventional methods of prognosis, which are based on the analysis of obstetric history, of demographic factors and of premonitory symptoms, are neither objective, nor sensitive, nor specific.

Many studies have been carried out in order to search for biochemical markers correlated with a threat of preterm delivery, which would allow reliable and rapid diagnosis able to be used to monitor patients at risk.

WO 2006/086731 discloses a method for assessing the risk of preterm delivery wherein the level of methyladenine, heptanedioic acid and N-acetyl glutamine is determined in a biological sample, preferably amniotic fluid, obtained from a pregnant subject. US2006/0127962 discloses a diagnostic assay for determining a biomarker indicative of intra amniotic inflammation in a sample of amniotic fluid wherein the biomarker is a calgranulin. Amniotic samples are obtained by amniocentesis, a method which in itself increases the risk of foetal loss by approximately 1 %.

WO01/88545 concerns a method for forecasting impending preterm delivery by detecting the presence or absence of IL-6 in a cervicovaginal secretion sample. WO 93/24836 relates to a method wherein the levels of defensins are determined in a cervicovaginal secretion sample from a pregnant patient in order to establish an increased risk of preterm delivery. Cervicovaginal secretion samples are not easily obtained and in many cultures vaginal fluid sampling would not readily be accepted as part of a routine screening assay.

Foetal fibronectin in vaginal fluid is also often used as a biomarker for imminent delivery. Such markers may predict delivery within a week or two, however, they are not indicative of preterm delivery for months in advance.

In order to be suitable as a screening assay, a method should be easy to perform, at low cost and without too much risk for the patient. It is also important that the method should have a low false positive rate (high specificity) so that patients who would deliver at term are not unnecessarily treated.

WO 2006/084109 discloses a method for identifying an increased risk of preterm delivery wherein an increase of salivary protease levels is determined. WO 96/12967 provides a method for the early detection of premature delivery comprising measuring the maternal level of corticotrophin-releasing hormone (CRH) in plasma or serum. When plasma CRH is high preterm birth is likely but a low level does not exclude preterm birth.

Despite of all these efforts, there is still a need for reliable biomarkers for the early detection or prediction that a given pregnancy may result in premature delivery.

### Summary of the invention

The present invention provides a method for identifying women with an increased risk of premature delivery comprising the steps of obtaining a biological sample from a pregnant patient after week 12 and prior to week 37 of pregnancy and in the biological sample, determining the level of at least one biomarker selected from the group consisting of inter-alpha trypsin inhibitor heavy chain H4, apolipoprotein B48 receptor, transferrin, alpha-1B-glycoprotein, antithrombin III and apolipoprotein A-IV precursor and comparing the level of the at least one biomarker with a level characteristic of pregnancies that proceed to term, wherein an abnormal level of said at least one biomarker is indicative for an increased risk of preterm delivery.

In contrast to most of the prior art methods, the method according to the invention is capable of predicting a preterm delivery months in advance, whereas the prior art provides assays that may be used when delivery is imminent, i.e. within a week or two.

The term abnormal level is meant to indicate either a decreased level of inter-alpha trypsin inhibitor heavy chain H4, apolipoprotein B48 receptor, transferrin or apolipoprotein A-IV precursor or an increased level of alpha-1B-glycoprotein or antithrombin III.

The invention also provides a method for preventing a preterm delivery comprising the steps of identifying women with an increased risk of premature delivery using a method according to the invention and treating women with an increased risk of preterm delivery with a tocolytic agent.

The invention also provides a method for improving the life expectancy of a newborn by ameliorating the effects of a preterm delivery, comprising the steps of identifying women with an increased risk of premature delivery using a method according to the invention and treating women with an increased risk of preterm delivery with a steroid or another effective medication in order to promote foetal lung maturation or to delay delivery with treatment such as administration of progesterone (da Fonseca EB, Bitter RE, Carvalho MH, Zugaib M. Prophylactic administration of progesterone by vaginal suppository to reduce the incidence of spontaneous preterm birth in women at increased risk: a randomized placebo-controlled double-blind study. Am J Obstet Gynecol. 2003; 188(2):419-424 and Meis PJ, Klebanoff M, Thom E, et al. Prevention of recurrent preterm delivery by 17 alpha-hydroxyprogesterone caproate. National Institute of Child Health and Human Development Maternal-Fetal Medicine Units Network. NEJM. 2003; 348(24):2379-85.)

The invention also provides a method for determining whether a given pregnancy is likely to proceed to full term, the method comprising the steps of obtaining a biological sample from a pregnant patient after week 12 and prior to week 37 of pregnancy and determining the level of at least one biomarker selected from the group consisting of inter-alpha trypsin inhibitor heavy chain H4, apolipoprotein B48 receptor, transferrin, alpha-1B-glycoprotein, antithrombin III and apolipoprotein A-IV precursor, comparing the level of the at least one biomarker with a level characteristic of pregnancies that proceed to term, wherein a normal level of said at least one biomarker is indicative for a pregnancy that will proceed to full term.

### Legend to the figure

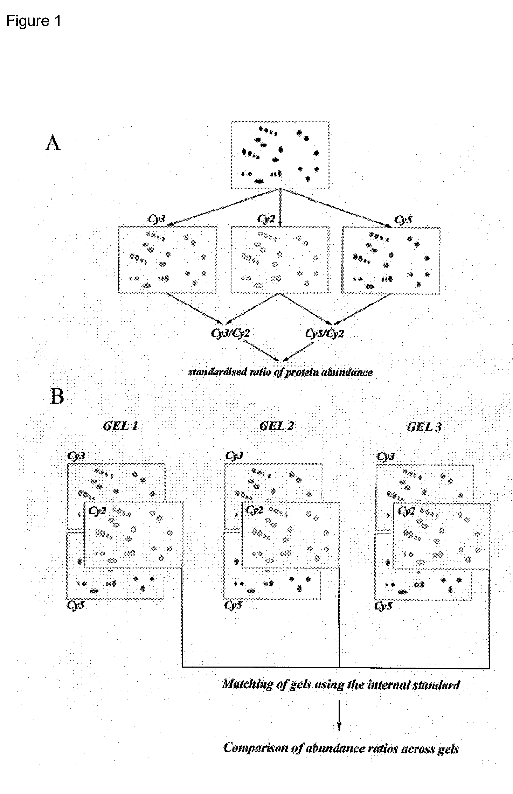

Figure 1. Statistical Analysis of 2D-DIGE Gels. (A) Following imaging, protein spots labelled with either Cy3 or Cy5 are standardised against the pooled internal control spot labelled with Cy2. Gel to gel variation is removed as the samples have all been resolved in the same gel. This is performed in the DIA module of DeCyderTM 2-D Differential Analysis Software. (B) The internal standard is also used for matching and comparing protein spot abundance across gels (performed by the DeCyderTM BVA module). This enables the statistical comparison of protein abundance changes in multiple samples.

### Detailed description of the invention

The present invention relates to a screening assay which provides an early, biochemical indication of increased risk of preterm delivery. The method can provide an indication of impending preterm delivery as early as three to four months prior to delivery, such as 2 or 1 month or 4, 3, 2 or 1 week. The method allows early intervention in the course of preterm delivery and provides an additional factor which can indicate those pregnancies at greatest risk.

The method comprises obtaining a biological sample from the pregnant patient after about week 12 of pregnancy and prior to about week 36 or 37 and determining the level of one or more biomarkers as disclosed herein in the sample. The presence of an elevated level of the biomarker in the sample indicates a patient who is at risk for preterm delivery.

The present method can determine impending delivery from very early in gestation through week 36 or 37. Deliveries prior to 20 weeks gestation are generally called spontaneous abortions rather than preterm deliveries. The present method can be used to detect spontaneous abortions (12 to 20 weeks gestation) and preterm deliveries (20 to 37 weeks gestation). Term pregnancies are from 37 to 40 weeks.

Premature delivery or preterm delivery or preterm birth are used interchangeably herein and are herein defined as birth after week 20 but before 37 completed weeks of pregnancy and may occur due to several different aetiologies.

The term normal pregnancy is meant to indicate a pregnancy that proceeds to term without the complication of preterm delivery. Preferably, a normal pregnancy is a pregnancy without any other complications, such as in particular preeclampsia or a growth retardation of the foetus.

A biological sample may actually be any sample obtained from a pregnant women, the results presented in the present specification are based on sera obtained from pregnant woman. One may also conceive to utilize other biological fluids such as amniotic fluid, vaginal excretions, saliva, and urine. Preferably, however, the biological sample is a blood, plasma or serum sample. It is a great advantage when a screening assay can be performed on a blood, serum or plasma sample, since these samples can be taken from a patient with minimal discomfort.

The levels of a particular biomarker provided herein are to be compared with the levels of that same biomarker in the same type of sample taken from a number of patients that experienced term delivery. Preferably those control samples are from a normal pregnancy of the same gestational period or age as the period or age of the patient screened for the risk of preterm delivery.

For an even higher precision of the method, the samples may be age matched.

It will be understood that the level of markers as disclosed herein can be measured directly or indirectly by a variety of different techniques. However, if the method is to be used as a screening assay, an immunological method is preferred. Antibodies against all the biomarkers provided herein are readily available and a person skilled in the art will know how to design and develop an immunological assay based on such antibodies. For some of the markers, even commercial assays are available.

Particularly preferred immunological methods are radioimmunoassays and enzyme-linked immunosorbent assays (ELISAs) because they are particularly amenable to routine laboratory practice.

A significant problem for premature babies is lung immaturity. Foetal lungs can be matured by the administration of beta methasone. However, if delivery is likely the beta methasone needs to be administered quickly in order to permit lung maturation to occur prior to delivery.

A method of determining that delivery is imminent and that consequently tocolytic therapy may not succeed can improve patient care by facilitating clinical decision making with regard to administration of agents such as steroids to mature foetal lungs. This treatment can be undertaken as a priority followed by a decision regarding persevering with tocolytic therapy. Hence, the invention relates to a method for ameliorating the effects of a preterm delivery for the newborn, comprising the steps of identifying women with an increased risk of premature delivery according to the invention and treating women with an increased risk of preterm delivery with a steroid or another effective medication in order to promote foetal lung maturation. Or preventing preterm birth in a woman at high risk for preterm birth by administering progesterone.

The methods as described herein may also be useful to confirm that any given pregnancy will proceed to term, if the level of the biomarker measured is within the normal range.

### Patients to be tested

The present method can be used on any pregnant woman following about 12 weeks gestation and prior to term pregnancies (week 36 or 37). In addition to screening any pregnant woman to determine whether delivery is imminent, the patients who are preferably screened are those patients with clinically intact membranes in a high risk category for preterm delivery, and more preferably, all those women whose pregnancies are not sufficiently advanced to ensure delivery of a healthy foetus. Ninety percent of the foetal morbidity and 100 percent of the foetal mortality associated with preterm delivery is for those foetuses delivered prior to 32 to 34 weeks gestation.

Therefore, 32 to 34 weeks gestation is an important cut-off for the health of the foetus, and preferably women whose pregnancies are at least about 12 weeks and prior to 34 weeks in gestation are tested.

In addition there are a large number of factors known to be associated with the risk of preterm delivery. Those factors include multiple foetus gestations; smoking, incompetent cervix; uterine anomalies; polyhydramnios; nulliparity; previous preterm rupture of membranes or preterm labor; preeclampsia; first trimester vaginal bleeding; little or no antenatal care; and symptoms such as abdominal pain, low backache, passage of cervical mucus and contractions.

Any pregnant woman at 12 or more weeks gestation with clinically intact membranes and having one or more risk factors for preterm delivery is preferably tested throughout the risk period; i.e., until about week 34 to 37.

Preferably, the method is used on samples taken in the second trimester of pregnancy. The levels of the biomarkers disclosed in this application may be measured in blood, preferably in serum or plasma.

Preferably, the samples should be tested immediately after preparation. Storage up to several days under appropriate conditions was shown not to affect the results, nevertheless, addition of protein stabilisers and protease inhibitors such as kallikrein inhibitors (PMSF) is recommended.

### Assay procedures

Abnormal levels of Inter-alpha trypsin Inhibitor heavy chain H4 (ITI H4) were found to be indicative of the diagnosis of preterm delivery. On average, patients with term delivery exhibited a level of ITI H4 that was about 2 times higher as compared to patients with preterm delivery.

Levels of ITI H4 were measured using two dimensional differential gel electrophoresis (2D-DIGE) as described in the examples. 2D-DIGE is a powerful tool to enable simultaneous visualization of relatively large portions of the proteome (Marouga et al., Anal. Bioanal. Chem. 2005. 382:669-78). For the routine determination of ITI H4 in the clinical laboratories, an immunological method is preferred. Such methods are further detailed in example 10.

The molecular cloning of ITI H4 has been described in Saguchi K. Et al., J. Biochem. 119: 898-905 (1996). This allows the expression of recombinant ITI H4 and the generation of monoclonal and polyclonal antibodies against this marker protein (Examples 8 and 9). Moreover, methods for the quantitative determination of inter-alpha trypsin inhibitors have been described. EP 0764849 A1 describes an immunological ELISA for the determination of ITI H1 and H2 whereas WO 01/63280 describes the development of a monoclonal antibody specific for ITI. Choi-Miura describes a quantitative ELISA for the measurement of IHRP in human plasma which is believed to be identical with ITI H4 (Biol. Pharm. Bull. 24; 214-217).

Abnormal levels of Apolipoprotein B48 receptor were also found to be indicative of the diagnosis of preterm delivery. On average, patients with term delivery exhibited a level of Apolipoprotein B48 receptor that was about 1.6 times higher as compared to patients with preterm delivery.

Levels of Apolipoprotein B48 receptor were measured using two dimensional differential gel electrophoresis (2D-DIGE) as described in the examples. For the routine determination of Apolipoprotein B48 receptor in the clinical laboratories, an immunological method is preferred. Such methods are described in example 10.

The gene encoding Apolipoprotein B48 receptor has been described in US 2003/0208060 and successful generation of specific antibodies has been disclosed therein.

Moreover, mouse antibodies against human Apolipoprotein B48 receptor (clone 2D7) are commercially available from Abnova Corporation Neihu district, Taipei 114 Taiwan ROC.

These antibodies are instrumental in designing a quantitative immunological assay for Apolipoprotein B48 receptor as further detailed in example 10.

Abnormal levels of transferrin were also found to be indicative of the diagnosis of preterm delivery. On average, patients with term delivery exhibited a level of transferrin that was about 1.4 times higher as compared to patients with preterm delivery.

Levels of transferrin were measured using two dimensional differential gel electrophoresis (2D-DIGE) as described in the examples. For the routine determination of transferrin in the clinical laboratories, an immunological method is preferred. Such methods are described in example 10.

Immunological assays for human transferrin are commercially available. An ELISA kit can be purchased at Bethyl Laboratories Montgomery, Texas or from Agdia Inc., Alpco Diagnostics, American Research products Inc, Biomeda Corp., or Kamiya Biomedical Company.

Abnormal levels of alpha-1B glycoprotein were also found to be indicative of the diagnosis of preterm delivery. On average, patients with preterm delivery exhibited a level of alpha-1B glycoprotein that was about 1.4 times higher as compared to patients with term delivery.

Levels of alpha-1B glycoprotein were measured using two dimensional differential gel electrophoresis (2D-DIGE) as described in the examples. For the routine determination of alpha-1B glycoprotein in the clinical laboratories, an immunological method is preferred. Such methods are described in example 10.

The gene encoding alpha-1B glycoprotein has been described in Strausberg R.L. et al Proc. Natl. Acad. Sci. U.S.A. 99:16899-16903(2002), thus allowing the production of specific antibodies. Moreover, rabbit antibodies against human alpha-1B glycoprotein are commercially available from Genway Biotech Inc in San Diego and from Aviva Systems Biology San Diego.

Abnormal levels of antithrombin III were also found to be indicative of the diagnosis of preterm delivery. On average, patients with preterm delivery exhibited a level of antithrombin III that was about 1.7 times higher as compared to patients with term delivery.

Levels of antithrombin III were measured using two dimensional differential gel electrophoresis (2D-DIGE) as described in the examples. For the routine determination of antithrombin III in the clinical laboratories, an immunological method is preferred. Such methods are described in example 10.

Mouse monoclonal antibodies against human antithrombin III are commercially available from AntibodyShop A/S in Gentofte, Denmark

Abnormal levels of apolipoprotein A-IV precursor were also found to be indicative of the diagnosis of preterm delivery. On average, patients with term delivery exhibited a level of apolipoprotein A-IV precursor that was about 1.8 times higher as compared to patients with preterm delivery.

Levels of apolipoprotein A-IV precursor were measured using two dimensional differential gel electrophoresis (2D-DIGE) as described in the examples. For the routine determination of apolipoprotein A-IV precursor in the clinical laboratories, an immunological method is preferred. Such methods are described in example 10.

Also, a quantitative ELISA for human apolipoprotein A-IV precursor has been described (Kondo et al., J. lipid Research 30, 939-944, 1989).

### Interpretation of results

Current biomarkers for the determination of the risk of preterm delivery suffer from the drawback of a low sensitivity. The markers identified herein were found to be remarkably sensitive, at an excellent specificity. When the lowest measured value of the normal (term delivery) samples was taken as the cut-off value, the method employing anti-thrombin III was found to be 100% specific and 100% sensitive (table 3).

The values obtained for Inter-alpha trypsin Inhibitor heavy chain H4 varied remarkably within the normal (term delivery) group as well as within the preterm delivery group (tables 1 and 2). At an average standardised abundance of 1.84, the standard deviation of 6 samples was 0.97. Due to this high variability, the sensitivity of this biomarker was the lowest among the group of 6 markers disclosed herein. Nevertheless, Inter-alpha trypsin Inhibitor heavy chain H4 is considered a valuable and specific marker for preterm delivery since the average standardised abundance of the normal term delivery group is about twice the value of the preterm group. The sensitivity of this marker could be remarkably increased, even up to 100%, if a cut-off value was used such that the specificity of the assay was only slightly decreased, i.e. in the order of 80%.

Antithrombin III was found to be the most sensitive marker. It exhibited a specificity and sensitivity of 100% if the cut-off value was properly chosen. (Table 1).

Apolipoprotein B48 receptor and alpha-1 B-glycoprotein were found to have a sensitivity of 80% when the cut-off values were chosen in such a way that the specificity of the assay would remain at 100% (table 3). A sensitivity of 100% could be reached for both markers at 80% specificity at appropriately chosen cut-off values.

In combination assays wherein several of the markers presented herein were combined, an even more sensitive method could be obtained. This is completely in line with the aetiology of preterm delivery as it is usually considered to have multiple causes.

Almost any combination of two markers chosen from the six markers presented herein, resulted in a method with an increased sensitivity. Hence, the invention relates to a method as described above, wherein at least two biomarkers were used, selected from the group consisting of inter-alpha trypsin inhibitor heavy chain H4, apolipoprotein B48 receptor, transferrin, alpha-1B-glycoprotein, antithrombin III and apolipoprotein A-IV precursor.

Particularly useful methods were those wherein at least two markers markers selected from the group consisting of apolipoprotein B48 receptor, alpha-1B-glycoprotein, apolipoprotein A-IV precursor and antithrombin III were combined. Although it is evident from the results presented herein that any combination of biomarkers may increase the sensitivity of the method, in particular the following combinations provided complementary results: inter-alpha trypsin inhibitor heavy chain H4 plus transferrin, inter-alpha trypsin inhibitor heavy chain H4 plus apolipoprotein A-IV precursor, apolipoprotein B48 receptor plus alpha-1B-glycoprotein, apolipoprotein B48 receptor plus transferrin, apolipoprotein B48 receptor plus alpha-1B-glycoprotein, apolipoprotein B48 receptor plus antithrombin III, transferrin plus alpha-1B-glycoprotein, transferrin plus antithrombin III, alpha-1B-glycoprotein plus antithrombin III, alpha-1B-glycoprotein plus apolipoprotein A-IV precursor and antithrombin III plus apolipoprotein A-IV precursor.

Sensitivity of the method could even be further improved when three, four, five or even all six biomarkers were used in a method according to the invention. Even when the cut-off value of the assays was chosen at the average normal value plus or minus two times the standard deviation of the normal population, a combination assay could be created that was 100% specific and 100% specific. Even more importantly, it was found that on average two markers were positive for each of the six samples obtained from the preterm delivery patients (table 2). In fact the same results were obtained when using only four out of the six markers (table 2). The only sample (#1) that was positive for only one marker (Alpha-1B- Glycoprotein) was not analysed against antithrombin III and Apolipoprotein A IV precursor because of a technical failure of the 2D gel analysis.

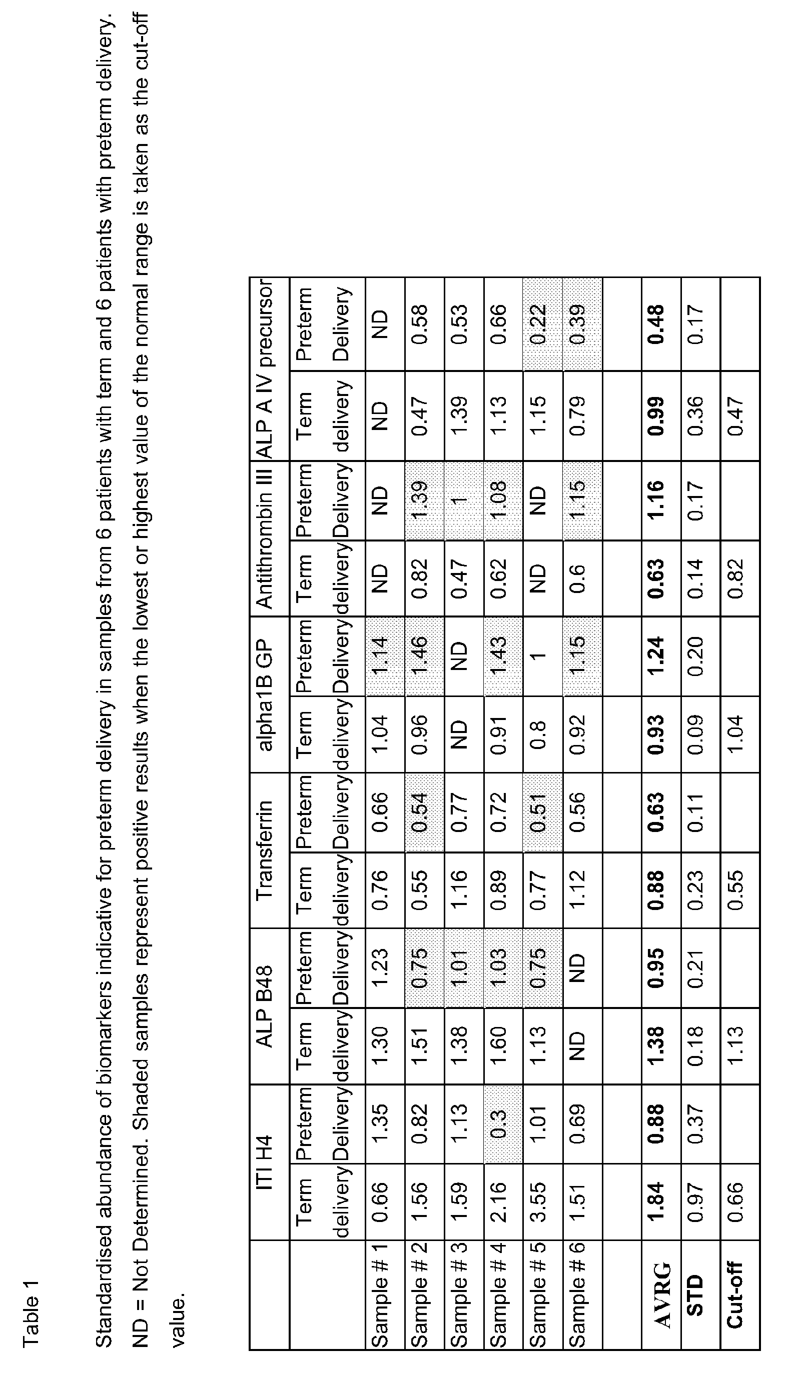

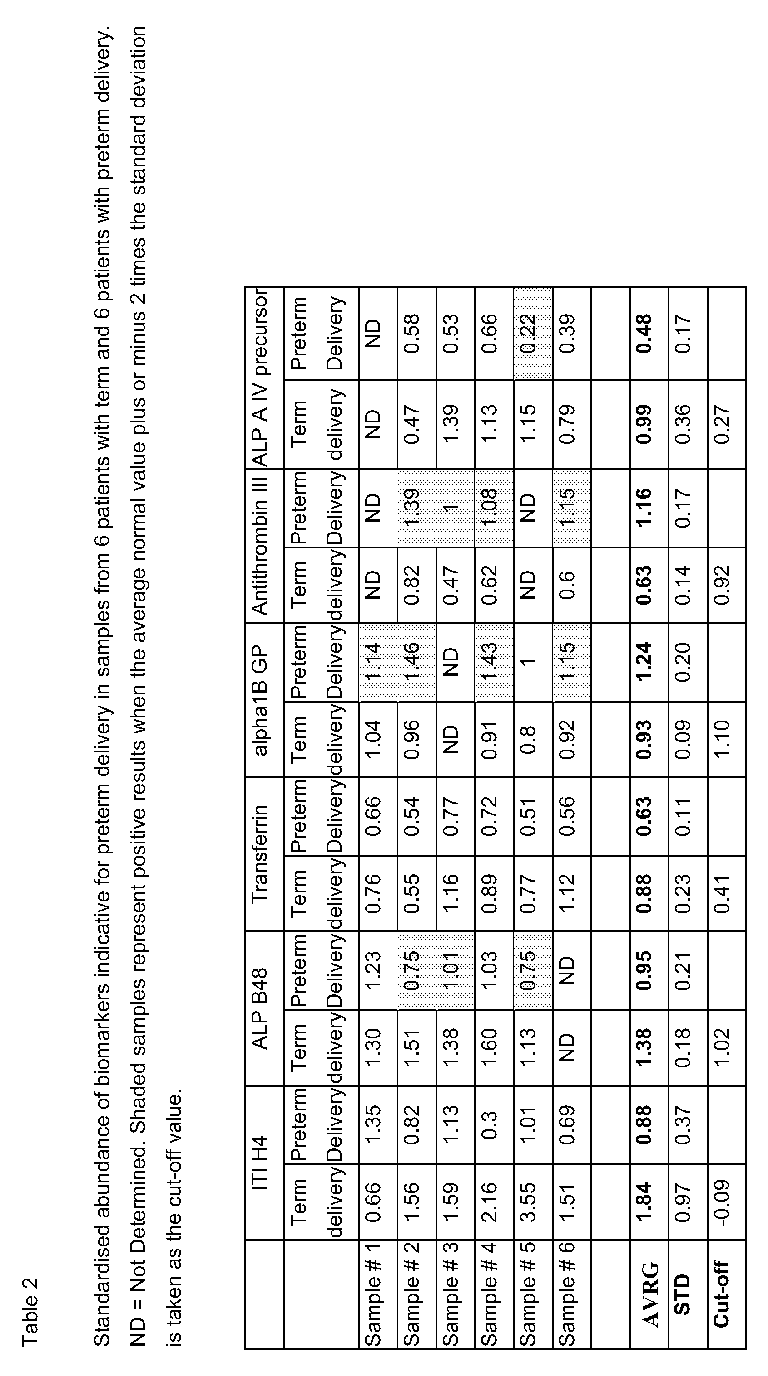

**Table 3.**

| Sensitivity, specificity, positive predictive value and negative predictive value of biomarkers for preterm delivery. Cut-off value = lowest/highest normal value. | | | | | | | | |
|---|---|---|---|---|---|---|---|---|
| Biomarker | False positive | False negative | True positive | True negative | Specificity [%] | Sensitivity [%] | PPV [%] | NPV [%] |
| Inter-alpha trypsin inhibitor heavy chain H4 | 0 | 5 | 1 | 6 | 100 | 17 | 100 | 55 |
| Apolipoprotein B48 receptor | 0 | 1 | 4 | 5 | 100 | 80 | 100 | 83 |
| Transferrin | 0 | 4 | 2 | 6 | 100 | 33 | 100 | 60 |
| Alpha-1 B-glycoprotein | 0 | 1 | 4 | 5 | 100 | 80 | 100 | 83 |
| Antithrombin III | 0 | 0 | 4 | 4 | 100 | 100 | 100 | 100 |
| Apolipoprotein A-IV precursor | 0 | 4 | 2 | 6 | 100 | 33 | 100 | 60 |

### Examples

### Example 1: Plasma samples

Samples were obtained prospectively from pregnant women who were followed until delivery and clinical conditions identified and monitored. Within a cohort of 500 women who experienced preterm birth (prior to 37 weeks) were identified. A group of normal pregnant women were also identified. Blood plasma samples from these two groups taken at 24 weeks of pregnancy were subjected to two dimensional difference gel electrophoresis (2D-DIGE) analysis. Using 2D-DIGE, the plasma protein profiles of 6 normal pregnant women were compared with plasma proteins profiles from 6 women who experienced preterm birth.

### Example 2: Depletion of HSA and IgG and Preparation of Blood Proteins

Human Serum Albumin (HSA) and gamma-immunoglobulin (IgG) account for up to 60% of total blood protein content. This is problematic for protein-based studies of blood because these proteins can mask and prevent the analysis of lower abundance proteins. Samples were depleted of HSA and IgG using the Albumin and IgG Removal Kit (GE Healthcare, Piscataway, NJ, USA) according to the manufacturer's protocol. In brief, extracted samples were incubated with an anti-HSA/anti-IgG resin for 30mins at room temperature. Unbound proteins were separated from the absorption matrix using a mini-spin cartridge and centrifuged at 1000rpm for 2mins. To concentrate protein and remove excess salts, the HSA/IgG depleted samples were precipitated by adding 4 volumes of ice-cold acetone to each sample. The solution was then incubated for 3 hrs at -20°C and then centrifuged at 13000rpm for 15mins at 4°C. Samples were resuspended in 50µl of extraction buffer (7M urea, 2M thiourea, 30mM Tris, 4% w/v CHAPS, pH 8.5). Protein concentration estimation was performed using the 2-D Quant Kit (GE Healthcare).

### Example 3: Fluorescent Cy-dye labelling of samples

Prior to labelling, cyanine dyes Cy2, 3 and 5 (GE Healthcare) were reconstituted in anhydrous DMF. 50µg protein from normal spontaneous labour and the preterm labour group (PTL) were labelled with 400pM of either Cy3 or Cy5 dye on ice for 30mins in the dark. Half of each sample population was labelled with Cy3 and the other with Cy5 to account for any dye binding bias. An internal pooled sample was created by mixing 25µg of each sample and labelling as above with Cy2. Dye labelling reactions were stopped by adding 10mM lysine and incubating for 10mins on ice in the dark. Cy3 and Cy5-labelled samples were mixed with the Cy2-labelled pooled control and made up to a volume of 450µl with rehydration buffer (7M urea, 2M thiourea, 4% w/v CHAPS, 2mg/mL DTT and 1% v/v pH3-10 immobilised pH gradient (IPG) buffer).

### Example 4: Two-dimensional gel electrophoresis

In-gel rehydration of IPG strips (non linear, pH3-10, 24cm; GE Healthcare) was performed with the Cy-labelled samples overnight for 12hrs at room temperature under mineral oil (Sigma, St. Louis, MO, USA). Isoelectric focusing was performed using a Multiphor II unit (GE Healthcare) for a total of 58800Vhrs using the following parameters; hold at 300V for 900Vhrs; ramp and hold at 1000V for 3900Vhrs; ramp and hold to 8000V for 13000Vhrs; hold at 8000V for 41000Vhrs. After focusing strips were equilibrated and reduced in equilibration buffer (30% v/v glycerol, 2% w/v SDS, 7M urea, trace bromophenol blue) with 0.5% DTT for 15mins at room temperature and then in equilibration buffer containing 4.5% iodacetamide for 15mins at room temperature. Equilibrated strips were applied on homogeneous 10% acrylamide gels (25.5 x 20.5cm) cast in low-fluorescence plates using the EttanTM DALTsix Electrophoresis Casting System (GE Healthcare). This enabled the production of six simultaneously-cast gels using the same batch of acrylamide gel stock solution for each gel. Second-dimensional electrophoretic separation was carried out at 2.5W/gel constant power for 30mins then 100W (total) constant power using a peltier-cooled Ettan DALT II electrophoresis unit (GE Healthcare) until the bromophenol dye front reached the bottom of the gel.

### Example 5: Imaging and Analysis of 2D-DIGE gels

Fluorescence image acquisition was performed using the Typhoon 9400 Variable Mode Imager (GE Healthcare). Each gel was scanned at the following excitation/emission wavelengths; 480/530nm (Cy2), 520/590nm (Cy3) and 620/680nm (Cy5). Gel analysis was conducted using the DeCyder V5.0 Biological Variation software module (GE Healthcare). This enabled spot matching between gels and also to quantify and standardise protein spot data. Spot matching was validated by manual inspection of gel spots. Statistically significant protein abundance changes were identified via Student's T-test. Proteins that were present in either all gels or at least in the spontaneous normal labour group with a p-value of p<0.05 or less were chosen as proteins for identification.

### Example 6: Utilisation of the internal standard

The primary advantage of 2D-DIGE over other proteomic techniques is the inclusion of an internal standard control. This allows to compensate for variation within gels and across gels in an experimental series. The internal control was created by pooling equal quantities of all samples to be assessed in each experiment. The pool was labelled with one of the Cy fluorophores (Cy2) and applied to every gel in the experiment containing Cy3 and Cy5 labelled samples. This means that every individual protein spot on the gel was be represented by the Cy2-labelled internal control. Therefore each protein spot could be standardised to its own control spot as well as across all gels in the experiment (Figure 1). In this way gel-to-gel variation was eliminated. This also allowed for the discrimination between system variation and true biological variation.

### Example 7: Protein sequencing

Two sequencing gels were prepared by performing 2D-PAGE on 500µg and 1000µg of pooled myometrium proteins. The gels were fixed in 50% methanol, 7% acetic acid for 30mins at room temperature and then stained with SYPRO Ruby (Invitrogen, California, USA) overnight also at room temperature. The gels were then washed in 10% methanol, 7% acetic acid and briefly rinsed in distilled water before being visualised using a UV illuminator. Proteins of interest were excised from the gels and destained in 50mM ammonium bicarbonate in 50% v/v methanol for 3 washes of 40mins each. Gel plugs were then dried at 37°C for 2hrs and then incubated in 40ng/µl trypsin (Promega, Madison, WI, USA) in 20mM ammonium bicarbonate at 37°C for 3 hours. Protein sequencing was performed via matrix-assisted laser desorption time-of-flight mass spectrometry (MALDI-ToF) using the Ettan MALDI-ToF Pro (GE Healthcare). Approximately 1µl tryptic peptide was mixed with 1µl a-cyano-4-hydroxycinnamic acid matrix (5 mg/ml in 50% acetonitrile, 0.1 % trifluroacetic acid) and 1µl of this mixture was spotted in duplicate onto the Maldi target tray. Peptide mass fingerprinting was performed alongside peptide standards (company) in reflectron mode. The external standard was use to calibrate the mass spectrometer prior to acquisition of sample MS data acquisition. Sequence data was used to interrogate the Swiss-Prot and NCBInr databases. As a further control measure, the isoelectric point and molecular weight of positively identified proteins were checked against the region of the pick gel they were excised from.

The following procedures are constructively reduced to practice and therefore described in the present tense, as opposed to the previous examples which were carried out in the laboratory and which are therefore set forth in the past tense.

### Example 8: Purification of polypeptides useful in the Invention

The polypeptide markers that are useful in the invention can be produced by first cloning the corresponding polynucleotides into a mammalian expression vector using established protocols that are familiar to those in skill of the art. In brief, polynucleotides of the invention are amplified and isolated via agarose gel electrophoresis and gel excision. The polynucleotides are subsequently cloned into appropriate vectors to facilitate in-frame cloning. Proteins may for instance be expressed with a polyhistidine metal-binding tag. The vectors with polynucleotides of the invention inserted can then be transfected into a mammalian cell line like COS-7 for high-level expression of the corresponding polypeptides.

The primary nucleotide sequence of all markers useful in the invention may be obtained from Genbank (table 4).

The polypeptides useful in the invention that are produced by the transfected mammalian cell line can then be purified according to established protocols that are familiar to those of skill in the art, for example using the Ni-NTA Magnetic Agarose Beads protein purification protocol, Qiagen, Valencia, Calif. In brief, assays utilizing Ni-NTA Magnetic Beads involve capture of the His-tagged protein-from a cell lysate or a purified-protein solution-followed by washing, binding of interaction partners, further washing, and finally elution of the interacting partner from the still immobilized His-tagged protein or elution of the interacting-partner-His-tagged-protein complex. Between each step, the beads are collected by attracting them to the side of the vessel, after placing near a magnet for 30-60 seconds. Purification procedures may even use crude cell extracts for binding of His-tagged protein.

The resulting purified polypeptides of the invention are then quantified, lyophilized and stored at 4 degrees Celcius or below, and may be used for standards in diagnostic kits or for the generation of antibodies.

### Example 9: Production of Specific Antibodies for the Polypeptides of the Invention

Polyclonal antibodies are produced by DNA vaccination or by injection of peptide antigens into rabbits or other hosts. An animal, such as a rabbit, is immunized with a peptide advantageously conjugated to a carrier protein, such as BSA (bovine serum albumin) or KLH (keyhole limpet hemocyanin). The rabbit is initially immunized with conjugated peptide in complete Freund's adjuvant, followed by a booster shot every two weeks with injections of conjugated peptide in incomplete Freund's adjuvant. Antibodies specific for a polypeptide of the invention are affinity purified from rabbit serum using peptides corresponding to the polypeptides of the invention, for instance coupled to Affi-Gel 10 (Bio-Rad), and stored in phosphate-buffered saline with 0.1% sodium azide. To determine that the polyclonal antibodies are specific for a polypeptide of the invention, an expression vector encoding a polypeptide of the invention is introduced into mammalian cells. Western blot analysis of protein extracts of non-transfected cells and the cells containing a polypeptide of the invention is performed using the polyclonal antibody sample as the primary antibody and a horseradish peroxidase-labeled anti-rabbit antibody as the secondary antibody. Detection of a band in the cells containing a polypeptide of the invention and lack thereof in the control cells indicates that the polyclonal antibodies are specific for the polypeptide of the invention in question.

Monoclonal antibodies are produced by injecting mice with a peptide derived from a marker polypeptide of the invention, with or without adjuvant. Subsequently, the mouse is boosted every 2 weeks until an appropriate immune response has been identified (typically 1-6 months), at which point the spleen is removed. The spleen is minced to release splenocytes, which are fused (in the presence of polyethylene glycol) with murine myeloma cells. The resulting cells (hybridomas) are grown in culture and selected for antibody production by clonal selection. The antibodies are secreted into the culture supernatant, facilitating the screening process, such as screening by an enzyme-linked immunosorbent assay (ELISA). Alternatively, humanized monoclonal antibodies are produced either by engineering a chimeric murine/human monoclonal antibody in which the murine-specific antibody regions are replaced by the human counterparts and produced in mammalian cells, or by using transgenic "knock out" mice in which the native antibody genes have been replaced by human antibody genes and immunizing the transgenic mice as described above.

Suitable antibodies may also be commercially obtained as described above.

### Example 10: Diagnostic Kit for markers according to the Invention

A diagnostic kit can be made in order to determine the levels of selected polypeptides of the invention that are present in samples obtained from a patient who is undergoing pregnancy. Such samples may convenieintly be from blood, blood plasma, blood serum, amniotic fluid, saliva or urine.

A diagnostic kit may comprise some or all of the following components: 1) one or more standards comprised of one or more polypeptides of the invention, prepared as described in example 8; 2) an antibody or a plurality of antibodies that are specific for the polypeptides of the invention that are being tested by the kit, prepared as described in example 9; 3) written instructions; 4) diluents for samples and the standards; 5) a wash buffer; 6) color reagents; 7) stop solution; and 8) an antibody carrier such as polystyrene beads or a lateral flow device or a microplate with bound antibody.

An example of such a kit is a quantificative ELISA (enzyme-linked immunosorbent assay) that determines the concentration or concentrations of a polypeptide or polypeptides according to the invention. The principle of the assay is to use the quantitative sandwich enzyme immunoassay technique where a monoclonal or polyclonal antibody selective for binding a polypeptide of the invention is pre-coated onto a carrier such as a microplate into its wells. The standards and sample are then pipetted into the wells and any of the polypeptide of the invention that is present is bound to this immobilized antibody. Next, the wells are washed with washing buffer, and an enzyme-linked monoclonal or polyclonal antibody that is specific for the polypeptide of the invention is added to the wells. Washing again is performed, then a substrate solution is added to the wells. Color subsequently develops in proportion to the amount of polypeptide of the invention that is bound in the first step. The color development is stopped using a stop solution, and the intensity of the color is measured by a microplate reader.

Alternatively, a lateral flow assay may be developed. Such lateral flow assays have the potential to be a cost-effective, fast, simple, and sensitive method, for instance for on-site screening assays. The principle is well known to the skilled person and consist essentially of a carrier that allows a lateral flow to occur wherein either the sample or the detection reagent is displaced form one location on the carrier to another. There are many formats of lateral flow assays suitable for the method according to the invention, the skilled person will know how to choose and optimize a particular format.

### Example 11: Diagnostic Chip for Polypeptides of the Invention

The levels of polypeptides of the invention in biological samples can also be determined through the use of ProteinChip technology (Wright Jr., et al. Expert Review of Molecular Diagnositcs 2002, Vol. 2 pp. 549-63 hereby incorporated herein by reference). This process includes the additon of a few microliters of sample onto the ProteinChip surface, which has both chemical (e.g., anionic, catioinic, hydrophobic, hydrophilic, metal) and biochemical (antibody, receptor, DNA, enzyme probes attached to its surface. Subsequently, binding occurs between the polypeptides of the invention and their respective probes, and the chip is washed at least three times in order to elute unbound protein, lipids, salts, and other substances. Next, an energy-absorbent molecule (EAM) such as sinapinic acid is added to the chip, and the chip is placed into a ProteinChip Reader which measures the molecular mass of the bound polypeptides. Peaks are subsequently produced from the ProteinChip Reader that are used to quantify the amount of polypeptides of the invention in the sample.

**Table 4: List of high molecular weight proteins identified as biomarkers of preterm delivery.**

| Protein ID | Swiss Prot accession # and entry name | Synonyms | MW of unprocessed precurser (KDa) | pl | Peptides Sequenced | % sequence coverage |
|---|---|---|---|---|---|---|
| Inter-alpha-trypsin heavy chain H4 | Q14624, ITIH4_HUMAN | ITI heavy chain H4, Inter-alpha-inhibitor heavy chain 4, Inter-alpha-trypsin inhibitor family, heavy chain-related protein, IHRP,Plasma kallikrein sensitive glycoprotein 120, PK-120, GP120 | 103.358 | 6.5 | 7 | 8.8 |
| Apolipoprotein B48 receptor | Q9NPJ9, Q9NPJ9_HUMAN | | 114.835 | 4.4 | 6 | 8.7 |
| Transferrin | P02787 TRFE_HUMAN | serotransferrin, siderophilin, beta-1-metal-binding globulin | 77.05 | 6.9 | 21 | 26.1 |
| Alpha-1B-glycoprotein | P04217 AIBG_HUMAN | | 54.273 | 5.6 | 5 | 12.3 |
| Antithrombin III | P01008 ANT3_HUMAN | ATIII | 52.602 | 6.3 | 6 | 11.9 |
| Apolipoprotein-A-IV precursor | P06727 APOA4_HUMAN | Apo-AIV, ApoA-IV | 45.399 | 5.3 | 14 | 36.6 |

## Claims

1. A method for identifying women with an increased risk of premature delivery comprising the steps of
a) Obtaining a biological sample from a pregnant patient after week 12 and prior to week 37 of pregnancy
b) In the biological sample, determining the level of at least one biomarker selected from the group consisting of inter-alpha trypsin inhibitor heavy chain H4, apolipoprotein B48 receptor, transferrin, alpha-1 B-glycoprotein, antithrombin III and apolipoprotein A-IV precursor,
c) Comparing the level of the at least one biomarker with a level characteristic of normal pregnancy such as a pregnancy that proceeds to term,
wherein an abnormal level of said at least one biomarker is indicative for an increased risk of preterm delivery.

2. A method according to claim 1 wherein the abnormal level is either a decreased level of inter-alpha trypsin inhibitor heavy chain H4, apolipoprotein B48 receptor, transferrin or apolipoprotein A-IV precursor or an increased level of alpha-1 B-glycoprotein or antithrombin III.

3. A method according to claim 1 or 2 which is an immunological method.

4. A method according to claim 3 which is a radioimmunoassay or an ELISA or a lateral flow assay.

5. A method according to claims 1 - 4 wherein the at least one biomarker is selected from the group consisting of apolipoprotein B48 receptor, alpha-1 B-glycoprotein and antithrombin III.

6. A method according to claims 1 - 5 wherein the biological sample is blood or plasma or serum.

7. A method for preventing a preterm delivery comprising the steps of identifying women with an increased risk of premature delivery according to claims 1 - 6 and treating women with an increased risk of preterm delivery with a tocolytic agent.

8. A method for ameliorating the effects of a preterm delivery for the newborn, comprising the steps of identifying women with an increased risk of premature delivery according to claims 1 - 6 and treating women with an increased risk of preterm delivery with a steroid or another effective medication in order to promote foetal lung maturation.

9. A method for determining whether a given pregnancy is likely to proceed to full term, the method comprising the steps of
a) Obtaining a biological sample from a pregnant patient after week 12 and prior to week 37 of pregnancy
b) In the biological sample, determining the level of at least one biomarker selected from the group consisting of inter-alpha trypsin inhibitor heavy chain H4, apolipoprotein B48 receptor, transferrin, alpha-1 B-glycoprotein, antithrombin III and apolipoprotein A-IV precursor,
c) Comparing the level of the at least one biomarker with a level characteristic of pregnancies that proceed to term,
wherein a normal level of said at least one biomarker is indicative for a pregnancy that will proceed to full term.
